# EUROPÄISCHE PATENTSCHRIFT

(11) **EP 2 034 817 B1**
(45) Veröffentlichungstag und Bekanntmachung des Hinweises auf die Patenterteilung: **20.04.2011**
(21) Anmeldenummer: 07764846.7
(22) Anmeldetag: 25.06.2007
(51) Int. Cl.: A01M 1/20, A24F 25/00, A61L 9/04, B65D 85/10

(54) **Verfahren zur Aromatisierung von zellulosehaltigen, Tabak enthaltenden Produkten und System hierfür**
Method of aromatising products comprising tobacco and cellulose and corresponding system
Procédé d'aromatisation d'un produit contenant du tabac et de cellulose, et système correspondant

(30) Priorität: 24.06.2006 DE 102006029092
(43) Veröffentlichungstag der Anmeldung: 18.03.2009
(60) Teilanmeldung: 10014603.4
(73) Patentinhaber: Bell Flavors & Fragrances Duft und Aroma GmbH, 04205 Leipzig/Militz (DE)
(72) Erfinder: HUPPERT, Hans-Jürgen, 04207 Leipzig (DE); BERRIDO, Colin, Surrey GU19 5NU (GB)
(74) Vertreter: Bockhorni & Kollegen
(86) Internationale Anmeldenummer: PCT/EP2007/005618
(87) Internationale Veröffentlichungsnummer: WO 2007/147645

(56) Entgegenhaltungen:
- EP-A- 1 627 647
- WO-A-00/24434
- WO-A-02/09778
- DE-A1- 10 247 583
- DE-A1-102004 052 929
- DE-U1-202004 007 682
- US-A- 2 227 158
- US-A- 3 272 899
- US-A- 4 346 840
- US-A- 5 938 018

## Beschreibung

Die Erfindung betrifft ein Verfahren zur Präparierung, nämlich Aromatisierung von zellulosehaltigen Produkten, die Tabak aufweisen wie z. B. Zigaretten. Weiterhin betrifft die Erfindung auch ein System aus dem Mittel und zellulosehaltigen Produkten, die Tabak aufweisen.

Aus den Veröffentlichungen zum Stand der Technik ist eine Reihe von Möglichkeiten zur Aromatisierung von zellulosehaltigen Produkten bekannt. So beschreibt das deutsche Gebrauchsmuster DE 202 004 007 682 U1 mit Aroma ausgerüstete Tücher und Tüten, bei denen die Aromatisierung durch Besprühung, Benebelung, Begasung oder durch Tränkung der Produkte selbst oder der für ihre Herstellung verwendeten Ausgangsmaterialien mit Aroma freisetzenden Fluiden oder Lösungen erfolgt.

In ähnlicher Weise wird nach einem Verfahren zum Herstellen von aromatisiertem strangförmigem Rauchmaterial nach dem deutschen Patent DE 38 21 677 C2 ein flüssiges Medium, das einem aromatischen Stoff enthält, auf den Tabak aufgesprüht.

Diese vorgenannten Möglichkeiten zur Aromatisierung von zellulosehaltigen Produkten haben den Nachteil, dass zusätzliche und relativ komplizierte Verfahrensschritte während der Herstellung dieser Erzeugnisse erforderlich sind.

Aus der deutschen Offenlegungsschrift DE 44 03 018 A1 ist ferner ein Filter für Zigaretten, Pfeifen oder dergleichen bekannt, bei dem die Aromatisierung des Tabakrauches durch einen Aromastoff erfolgt, der in der Hülle für das Filtermaterial aufgenommen ist und den Rauch während des Passierens des Filters aromatisiert. Diese Form einer indirekten Aromatisierung hat den Nachteil, dass der Aromastoff, welcher in die Filterhülle eingebracht wurde, sich während der Lagerung der Filter bzw. der damit versehenen Rauchwaren relativ schnell verflüchtigt, so dass die Aromatisierung nur im frischen Zustand als ausreichend empfunden wird, jedoch schon nach kurzer Zeit nachlässt und kaum noch bemerkbar ist.

Weiterhin ist es bekannt, zellulosehaltige Produkte mit chemisch und/oder biologisch aktiven Substanzen (Wirkstoffen), wie Insektiziden und dgl., mittels Auf sprühen der Wirkstoffe zu versehen, um das Produkt über den Wirkstoff mit bestimmten Eigenschaften zu versehen. Auch hier besteht das Problem, dass diese Wirkstoffe in einem zusätzlichen Verfahrensschritt aufgebracht werden müssen und dass ein Nachlassen der Wirkung im Laufe der Zeit erfolgt.

Ein Verfahren nach dem Oberbegriff von Anspruch 1 ist aus der US 2 227 158 A bekannt.

Der Erfindung liegt deshalb die Aufgabe zugrunde, ein Verfahren zur Aromatisierung von zellulosehaltigen Produkten, die Tabak aufweisen, zu entwickeln, mit welchem eine gleichmäßige und über einen langen Zeitraum anhaltende Aromatisierung der Produkte gewährleistet werden kann, ohne dass für die Herstellung der Produkte zusätzliche und komplizierte Verfahrensschritte erforderlich sind.

Die Aufgabe wird erfindungsgemäß durch die Merkmale des Patentanspruchs 1 gelöst. Zweckmäßige Ausgestaltungen dieses Erfindungsgegenstandes bilden die Merkmale der Unteransprüche 2 bis 9.

Die Erfindung geht von der Erkenntnis aus, dass Produkte, insbesondere zellulosehaltige Produkte wesentlich effizienter und lang anhaltender mit solchen flüchtigen Substanzen versehen werden können, wenn diese flüchtigen Substanzen in einem festen Trägerstoff gebunden sind und mit dem Produkt unabhängig von dessen Herstellungsverfahren in Verbindung gebracht werden.

Dieses Verfahren der Aromatisierung von zellulosehaltigen Produkten, die Tabak aufweisen, ist nicht nur sehr effektiv, da die flüchtigen Substanzen über einen langen Zeitraum kontinuierlich freigesetzt werden, sondern es ist auch sehr kostengünstig, da so die Substanzen nicht dem Produkt selbst zugefügt werden müssen. Die flüchtigen Substanzen können in unmittelbarer Nähe der Produkte angeordnet sein, wobei sie so nah angeordnet sein müssen, dass eine ausreichende Menge der Substanzen zu dem Produkt gelangen kann. Natürlich können sie auch direkt an oder in dem Produkt (beispielsweise in einem gefalteten Handtuch) angeordnet werden. Dabei können die flüchtigen Substanzen auch mit dem Produkt in einer Produktverpackung angeordnet sein.

Erfindungswesentlich ist, dass der bei Normaltemperatur flüssige Geschmacks- oder Geruchsstoff in dem bei Normaltemperatur festen Trägerstoff eingekapselt ist, so dass der feste Trägerstoff die Geschmacks- oder Geruchsstoffe nicht nur speichert und Konserviert, sondern über die Lagerzeit Kontrolliert wieder abgibt.

Beispiele für solche festen Trägerstoffe sind in der DE 10 2004 052 929 A1 sowie der DE 102 47 583 A1 aufgezeigt, deren Inhalt hinsichtlich der Trägerstoffe und ihrer Verwendung hiermit vollumfänglich mit aufgenommen wird. Wichtig ist, dass es sich um Trägerstoffe handelt, die die Geschmacks- oder Geruchsstoffe binden. Es handelt sich also um einen bei Normaltemperatur flüssigen Geschmacks- oder Geruchsstoff der in dem bei Normaltemperatur festen Trägerstoff dadurch gebunden ist, dass der Geschmacks- oder Geruchsstoff an dem Trägerstoff ad- und/oder absorbiert ist. Der Duftstoff ist dabei vom Trägermaterial eingekapselt und wird langsam wieder freigegeben. Dies steht im Unterschied beispielsweise zu so genannten Duftbäumen für Automobile und dgl., bei denen ein Filz (poröser oder saugfähiger Träger) mit einem Duftstoff getränkt wurde.

Beispielsweise kann der feste Trägerstoff mit dem darin gebundenen mindestens einem Geschmacks- oder Geruchsstoff in kleinteiliger Form verwendet werden, etwa in Form von Pulver, Granulat, Chips oder Perlen. Andererseits kann der feste Trägerstoff mit dem darin gebundenen mindestens einem Geschmacks- oder Geruchsstoff auf ein Stützmaterial auf- oder in das Stützmaterial eingebracht werden, wobei bevorzugt ein Flachmaterial, vorzugsweise Papier, Karton, Kunststoff- oder Metallfolie, Keramik, Glas, Vlies oder Gewebe sowie ein gewebeartiges Produkt aus Stoff, Glas, Keramik, Metall oder Kunststofffasern verwendet wird. Das Stützmaterial kann auch ein durch Ur- oder Umformen erzeugtes oder aus mehreren Elementen zusammengesetztes Teil beliebiger Form sein. Dabei kann die Beschichtung des Stützmaterials mit dem festen Trägerstoff mit dem darin gebundenen mindestens einem Geschmacks- oder Geruchsstoff dadurch erfolgen, dass eine Schmelze des Trägerstoffes und des darin gebundenen mindestens einem Geschmacks- oder Geruchsstoffes auf das Stützmaterial auf- oder in das Stützmaterial eingebracht und anschließend durch Abkühlen verfestigt wird. Die Aufbringung kann dabei durch Beschichtung, Aufsprühen, Tränken oder Begießen erfolgen.

Vorzugsweise kann als Stützmaterial für die Auf- oder Einbringung des Trägerstoffes mit dem darin gebundenen mindestens einem Geschmacks- oder Geruchsstoff die Innenseite des Verpackungsmaterials selbst dienen.

Besonders bevorzugt ist der Trägerstoff gegenüber der Umgebung bzw. dem Produkt durch eine für den Trägerstoff bzw. den Trägerstoff und das Stützmaterial undurchdringbare Umhüllung versiegelt, das jedoch für die Aromatisierungsstoffe durchlässig ist. (Es reicht allerdings, wenn die Umhüllung nur in bestimmten, gewünschten Bereichen für den Geruchs- oder Geschmacksstoff durchlässig ist. Beispielsweise reicht eine einseitige Aufbringung eines durchlässigen Bereichs der Umhüllung auf einer Verpackung, die selbst als Stützmaterial dient, wenn die Verpackung für den Trägerstoff undurchlässig ist.) Dadurch wird der Aromatisierungsstoff durch die Umhüllung hindurch gelassen, um das Produkt zu aromatisieren, allerdings können weder der Trägerstoff noch das Stützmaterial mit dem Produkt in Berührung kommen, so dass auch eine besondere Sicherheit, vor allem für Kinder besteht. Diese Umhüllung kann Papier, Polyethylen, insbesondere modifiziertes Polyethylen, beispielsweise Ethylenethylazetat (EEA) oder Ethylenvinylazetat (EVA), normales PE, wie Low Density Polyethylen oder High Density Polyethylen, oder aus Silikonfolie umfassen oder vollständig aus diesen bestehen.

Schließlich kann vorgesehen sein, dass die Verpackung ein für den Geschmacks- oder Geruchsstoff undurchlässiges Material, wie Zellophan, umfasst, das insbesondere einstückig mit der Verpackung verbunden ist. Dadurch bleibt die Aromatisierung des Produkts länger erhalten und frisch. Dieses Material kann natürlich auch selbst die Verpackung bilden.

Flüchtige Substanzen im Rahmen der vorliegenden Erfindung sind also Aromastoffe, wie Geschmacks- und Geruchsstoffe.

Durch die Flüchtigkeit der Aromastoffe wird ein sinnliches Empfinden über entsprechende Rezeptoren (z.B. in der Nase) hervorgerufen. Beispielsweise bestehen Parfüme aus Kopf-, Herz- (oder Mittel-) und Basisnoten. Diese drei Noten enthalten unterschiedliche Geruchskomponenten, die sich in der Flüchtigkeit unterscheiden, wobei die Flüchtigkeit von der Basisnote über die Herznote zur Kopfnote ansteigt. Infolge dieser unterschiedlichen Flüchtigkeit verändert sich der Geruchseindruck bei längerer Lagerung. In gleicher Weise nimmt durch die Flüchtigkeit der Aromastoffe auch die Intensität ab.

Mit dem erfindungsgemäßen Verfahren werden nun diese Aromastoffe immer wieder aufgefrischt und dem Produkt vermittelt. Dies geschieht dadurch, dass der Feststoff die Aromastoffe nicht nur speichert und konserviert, sondern allmählich über die Lagerungszeit kontrolliert wieder abgibt.

Eine besonders vorteilhafte Weiterbildung des erfindungsgemäßen Verfahrens besteht darin, dass der Prozess der Aromatisierung durch die Eintragung von Energie beschleunigt wird. Als Energie kommt insbesondere elektromagnetische Strahlungen im Bereich des infraroten, des sichtbaren und des UV-Lichts in Betracht, aber auch Mikrowellenstrahlung und Ultraschall. Bevorzugt ist jedoch die Bestrahlung mit Mikrowellen- und Wärmestrahlung. Durch diese Bestrahlung wird eine beschleunigte Freisetzung der Aromastoffe aus dem Trägermaterial bewirkt.

Auf diese Weise können insbesondere Produkte, die nach Öffnen der Verpackung abgegeben werden und dann zu diesem Zeitpunkt auch optimal aromatisiert sein sollen, sehr schnell vorbereitet werden.

Zum Einsatz kommen können nun verschiedene Darreichungsformen des Trägerstoffs mit dem darin gebundenen Geruchs- oder Geschmacksstoff: zum einen eine kleinteilige Form als Granulat, wie Kugeln, Chips oder dgl., mit einem Außendurchmesser von bis 10 mm, vorzugsweise bis zu 5 mm, oder als Pulver im Bereich von 10 µm bis 100 µm, bevorzugt 25 µm bis 50 µm, oder zum anderen eine Filmform. Filmform heißt in diesem Zusammenhang, dass der Trägerstoff mit dem darin gebundenen Geruchs- oder Geschmacksstoff auf ein Stützmaterial auf- oder in das Stützmaterial eingebracht ist. Als Stützmaterial können u.a. Papier, Karton, Kunststoffe, Keramik oder Glas, Vlies oder Gewebe sowie gewebeartige Produkte aus Stoff, Glas, Keramik, Metall oder Kunststofffasern verwendet werden oder Laminate dieser Stoffe. Diese Stützmaterialien können als Flachmaterial oder als ein durch Ur- oder Umformen erzeugtes oder aus mehreren Elementen zusammengesetztes Teil beliebiger Form verwendet werden. Typischerweise sind solche Filmformen (Filme) bis zu 3 mm, vorzugsweise 2 mm dick. In diesem Zusammenhang wird auf die DE 10 2005 036 415 A1 verwiesen, deren Inhalt bezüglich der Filmform und ihrer Herstellung vollumfänglich in diese Beschreibung mit einbezogen wird.

Mit diesen Darreichungsformen sind nun bestimmte Eigenschaften verbunden.

Bei Granulaten sind die Geruchs- oder Geschmacksstoffe mit einem hohen Grade in dem Trägerstoff eingekapselt. Daher ist Energie notwendig, um diese Stoffe schnell wieder freizusetzen. Diese Stoffe werden aus Granulaten am besten bei Temperaturen von 50 °C bis 55 °C freigesetzt, allerdings besteht dabei die Gefahr, dass der Trägerstoff schmilzt und die Umgebung verunreinigt bzw. sogar schädigt. Andererseits können lose Pastillen jederzeit leicht verstreut und aus der bevorzugten Anwendungsumgebung verschleppt werden.

Bei der Filmform ist die Einkapselung nicht so stark wie bei Granulaten, da der Trägerstoff mit dem gebundenen Geruchs- oder Geschmacksstoff als dünne Filmschicht auf dem Stützmaterial angeordnet ist und daher die Geruchs- oder Geschmacksstoffe die Oberfläche schneller erreichen. Hiermit lässt sich ein schnelles Freisetzen schon bei Raumtemperatur erreichen, wobei allerdings auch Schäden beim direkten Kontakt der Umgebung mit der Filmform auftreten können (z.B. bei Berührung mit der Hand oder beim Kontakt mit anderen Gegenständen).

Das Pulver ist ein sehr feiner Puder, der beispielsweise durch kryogenes Vermahlen eines in fester Form vorliegenden Trägerstoffs mit dem gebundenen Geruchs- oder Geschmacksstoff unter flüssigem Stickstoff (oder Kohlendioxidgas oder Trockeneis) erzeugt wird. Auch andere Herstellungsmethoden sind anwendbar. Aufgrund seiner vergrößerten Oberfläche gibt Pulver die aufgenommenen Stoffe bei Raumtemperatur schneller ab als Granulate oder die Filmform. Aufgrund der geringen Partikelgröße ist jedoch die Handhabung erschwert und Verunreinigungen der Umgebung sind möglich.

Natürlich können diese Darreichungsformen auch miteinander kombiniert werden. Beispielsweise können in einer Verpackung verschiedene Darreichungsformen eingebracht sein, um jeweils verschiedene Geruchs- oder Geschmacksstoffe zu übertragen. Es können aber auch verschiedene Darreichungsformen eingebracht sein, die jeweils die selben Geruchs- oder Geschmacksstoffe enthalten.

Zur Überwindung dieser Probleme werden die vorgenannten oder auch andere denkbare Darreichungsformen erfindungsgemäß in einer Umhüllung eingeschlossen, die den Durchtritt des Trägermaterials (und des ggf. verwendeten Stützmaterials) verhindert und gleichzeitig für den Geruchs- oder Geschmacksstoff zumindest in einem Bereich durchlässig ist. Dadurch wird ein Austreten oder Inkontakttreten des Trägermaterials (und des ggf. verwendeten Stützmaterials) in die Umgebung und auch der direkte Kontakt verhindert.

Wichtig ist, dass die Umhüllung nur dort durchlässig sein muss, wo auch eine Durchlässigkeit für den Geruchs- oder Geschmacksstoff erforderlich ist. Die anderen Bereiche können für die Geruchs- oder Geschmacksstoffe undurchlässig sein, wobei sie jedoch ein Austreten oder Inkontakttreten des Trägermaterials (und des ggf. verwendeten Stützmaterials) in die Umgebung und auch den direkten Kontakt verhindern müssen.

Wenn also in jeden Raumwinkelbereich eine Durchlässigkeit erforderlich ist, dann muss die Umhüllung überall durchlässig sein für den Geruchs- oder Geschmacksstoff. Wenn nur für bestimmte Raumwinkelbereiche eine Durchlässigkeit erforderlich ist, dann ist die Umhüllung in diesen Bereichen durchlässig zu gestalten, in den anderen Bereichen reicht eine gänzlich undurchlässige Umhüllung, jedoch könnte die Umhüllung auch überall durchlässig gestaltet werden, was letztlich eine Kosten- und Designfrage ist.

Wenn beispielsweise der Trägerstoff mit einem Geruchsstoff auf einer Verpackung als Stützmaterial aufgebracht ist, dann reicht es, den für den Geruchsstoff durchlässigen Bereich der Umhüllung direkt auf dem Trägerstoff vorzusehen, da nur in diesen Bereichen eine Durchlässigkeit erforderlich ist, um in der Verpackung befindliche Produkte zu aromatisieren. Die Verpackung versiegelt als Teil der Umhüllung dann den Trägerstoff gegenüber der Umgebung der Verpackung und der durchlässige Bereich der Umhüllung versiegelt den Trägerstoff gegenüber dem Innenraum der Verpackung und somit gegenüber dem in der Verpackung aufgenommenen Produkt(en).

Mit dieser Umhüllung sind diese Mittel im Gebrauch sehr sicher, was vor allem unter dem Aspekt der Kindersicherung sehr wichtig ist.

Eine einfache Möglichkeit einer Umhüllung, die die Bedingungen an die Durchlässigkeit für diese Stoffe und die gleichzeitige Versiegelung des Trägerstoffs erfüllt, ist eine Hülle aus Papier, beispielsweise ein Teebeutelpapier, das zu einem Säckchen geformt ist. Dies ist ausreichend für Granulate und die Filmform, da das Papier diese Formen aufgrund ihrer Größe gut zurückhält. Es ist aber weniger gut für Pulver verwendbar aufgrund der geringen Partikelgröße der Pulverkörner. Außerdem besteht bei Teebeutelpapier auch die Gefahr eines Reißens aufgrund der geringen mechanischen Festigkeit. Wenn es aber darauf nicht ankommt, kann man durchaus Teebeutelpapier verwenden.

Alternativ können Polyethylen (PE), modifiziertes PE, beispielsweise Ethylenethylazetat (EEA) oder Ethylenvinylazetat (EVA), sowie Silikon zum Einsatz kommen.

PE ist ein weit verbreitetes, günstiges Material, das nicht polar ist und daher keine gute Durchlässigkeit für polare Substanzen, wie die in Geruchs- und Geschmacksstoffen üblicherweise vorkommenden Aldehyde, Ketone und Ester, besitzt. Dennoch wird aber eine geringe Durchlässigkeit bestehen aufgrund der in einer PE-Folie vorhanden kleinsten Zwischenräume zwischen den Polyolefinpartikeln.

PE ist in verschiedenen Varianten erhältlich, beispielsweise als LDPE (low density PE) und HDPE (high density PE). LDPE weist eine geringer gepackte Molekularstruktur auf als HDPE, die mehr Durchlass schon bei Raumtemperatur gestattet. Bei HDPE muss üblicherweise auf etwa 40 °C erhitzt werden, um eine ähnlich hohe Durchlässigkeit zu erzielen.

Alternativ kann eine PE-Folie für polare Substanzen dadurch durchlässig gemacht werden, dass ein nicht polarer Zusatzstoff (Lösungsmittel) verwendet wird, der die Substanzen durch die Folie "hindurchleitet". Das dahinter stehende Prinzip besteht darin, dass gleiche Stoffe gleiche Stoffe lösen. Da nämlich PE ein nicht polarer Stoff ist, werden beispielsweise nicht polare Kohlenwasserstoffe in das PE-Material aufgenommen und bewirken ein Aufquellen und damit eine Vergrößerung der Polyolefinpartikel, wodurch die zwischen den Polyolefinpartikeln befindlichen Zwischenräume vergrößert werden und eine größere Substanzmenge durch die PE-Folie hindurchtreten kann.

Als vorteilhaft für ein nicht polares Lösungsmittel hat sich ein iso-paraffinisches Lösungsmittel mit dem Markennamen Isopar von Exxon herausgestellt. Insbesondere Isopar L, das einen Flammpunkt von 62 °C aufweist, ist besonders gut geeignet. In Bezug auf die Gesamtmenge von Lösungsmittel und Geruchs- oder Geschmacksstoff sind Beimischungen dieses Lösungsmittels zu dem Geruchs- oder Geschmacksstoff von 0,5% bis 50% vorteilhaft, wobei bevorzugt 10% verwendet werden. Zu beachten ist allerdings, dass ein zu hoher Anteil an Lösungsmittel zu einer erhöhten Aufnahme in die Polyolefinpartikel und damit einem verstärkten Aufquellen der PE-Folie führt, wodurch Risse entstehen können. Andererseits führt die Verwendung von Lösungsmitteln mit geringerem Flammpunkt ebenfalls zu einer erhöhten Aufnahme und damit Anschwellen, so dass auch hier die Gefahr von Rissbildungen besteht.

Durch Zusatz polarer Lösungsmittel, insbesondere, wenn diese eine größere Polarität besitzen als die Geruchs- oder Geschmacksstoffe, kann die Durchlässigkeit durch die PE-Folie auch vermindert werden.

Eine weitere wichtige Einflussgröße für die Durchlässigkeit von PE-Folien ist die Foliendicke, denn je dicker die Folie ist, desto geringer ist der Durchlass. Vorteilhaft werden Schichtdicken von 0,1 mm bis 2 mm, bevorzugt von 0,2 mm bis 0,5 mm verwendet.

Somit ist deutlich geworden, dass die pro Zeit durchgelassene Substanzmenge sowohl vergrößert als auch verkleinert werden kann, wodurch leicht eine gezielte Anpassung an bestimmt Erfordernisse möglich ist.

Modifiziertes PE ist üblicherweise polarer als reines PE, so dass typische Geruchs- und Geschmacksstoffe passieren können ohne dass besondere Lösungsmittel erforderlich sind. Vielmehr wirkt der Stoff selbst wie ein polares Lösungsmittel. Bei diesen modifizierten PE steigt die Polarität mit zunehmendem Gehalt an Ethylazetat oder Vinylazetat, wodurch die Durchlässigkeit für polare Substanzen erhöht wird, jedoch die Festigkeit des modifizierten PE sinkt.

Modifiziertes PE ist allerdings teurer und daher nicht so weit verbreitet. Genau wie normale PE-Folien werden im Rahmen der vorliegenden Erfindung modifizierte PE-Folien bevorzugt im Temperaturbereich von 5 °C bis 80 °C, insbesondere bei 10 °C bis 40°C, eingesetzt.

Silikon-Folien werden vorteilhaft für Hochtemperaturanwendungen bis 200°C, vorzugsweise von 80 °C bis 120 °C, benutzt. Beispielsweise hat sich eine Folie mit dem Markennamen AltecSil der britischen Firma Altec für Geruchs- und Geschmacksstoffe bewährt.

Bevorzugt kommen Silikon-Folien mit einer Dicke von 0,1 mm bis 1 mm zum Einsatz, wobei die Durchlässigkeit mit geringerer Dicke ansteigt.

Selbständiger Schutz wird außerdem beansprucht für ein System aus solchen Mitteln und einem oder mehreren zellulosehaltigen Produkten, die Tabak aufweisen, gemäß Anspruch 10. Bevorzugt beinhaltet dieses System eine Verpackung, in der sowohl das Produkt als auch das Mittel aufgenommen ist, wobei die Verpackung vorteilhaft für Geruchs- oder Geschmacksstoffe undurchlässig ist, um so eine möglichst langanhaltende Aromatisierung zu ermöglichen.

Die Erfindung soll nachfolgend anhand von Ausführungsbeispielen unter Bezugnahme auf die Figuren näher erläutert werden. Dabei zeigen:
- Fig. 1: eine Zigarettenschachtel mit Aromastoffen, die in einem Feststoff- granulat gebunden sind,
- Fig. 2: eine Zigarettenschachtel mit Aromastoffen, die in einem Träger- stoff gebunden sind, der in und/oder an einem Stützmaterial ange- ordnet ist,
- Fig. 3: eine Zigarettenschachtel mit Aromastoffen, die in einem Träger- stoff gebunden sind, der in und/oder an einer Innenseite der Ziga- rettenschachtel gebunden ist,
- Fig. 4: ein Mittel zur Aromatisierung nach einer ersten Ausführungsform der Erfindung,
- Fig. 5: eine Vorrichtung zur Herstellung des Mittels nach Fig. 4,
- Fig. 6: ein Mittel zur Aromatisierung nach einer zweiten Ausführungsform der Erfindung,
- Fig. 7: ein Mittel zur Aromatisierung nach einer dritten Ausführungsform der Erfindung,
- Fig. 8: das Prinzip des Durchtritts polarer Substanzen durch eine nicht po- lare Umhüllung und
- Fig. 9: das Prinzip des Durchtritts polarer Substanzen, die in einem nicht polaren Lösungsmittel aufgenommen sind, durch eine nicht polare Umhüllung.

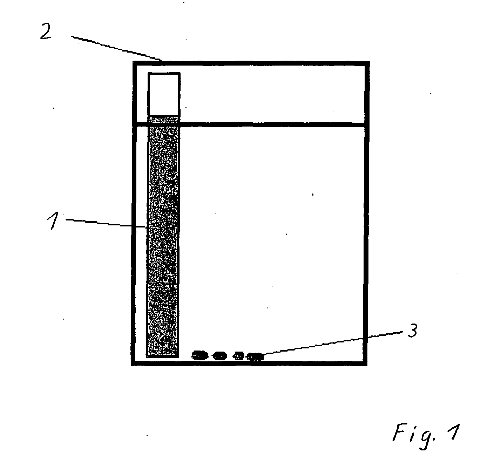

In einem ersten Ausführungsbeispiel, welches in Figur 1 rein schematisch dargestellt ist, wird die Erfindung anhand der Aromatisierung von Zigaretten in einer Verpackung beschrieben. Sowohl das Zigarettenpapier, der Filter und vor allem der Tabak stellen das zu aromatisierende zellulosehaltige Produkt 1 dar. In dem in Figur 1 dargestellten Ausführungsbeispiel erfolgt die Aromatisierung durch Einbringen des in einem festen Trägerstoff gebundenen Geschmacks- und/oder Geruchsstoffes 3 in kleinteiliger loser Form in die Verpackung 2. Der mindestens eine im festen Trägerstoff gebundene Geschmacks- und/oder Geruchsstoff 3 wird in der Verpackung 2 langsam und gleichmäßig freigesetzt und durchdringt das zellulosehaltige Produkt 1, d. h. das Zigarettenpapier, den Zigarettenfilter und den Tabak selbst und aromatisiert auf diese Weise das zellulosehaltige Produkt 1. Der in einem festen Trägerstoff gebundene mindestens eine Geschmacks- und/oder Geruchsstoff verbreitet sich innerhalb der Verpackung sofort nach dem Verschließen und aromatisiert die Zigaretten 1 gleichmäßig über einen langen Zeitraum.

Durch das erfindungsgemäße Verfahren ist es nicht notwendig, die zellulosehaltigen Materialien, d. h. im Ausführungsbeispiel die Zigaretten einschließlich des Zigarettenpapiers und des Filters während des Produktionsprozesses auf direktem Wege zu aromatisieren. Dadurch bleibt der Produktionsprozess völlig unbeeinflusst, und es sind keine zusätzlichen, kostenintensiven Verfahrensschritte im Produktionsablauf erforderlich.

Der hohe Anteil von bis zu 60 Gew.-% im Trägerstoff gebundenem Geschmacks- und/oder Geruchsstoff ermöglicht eine intensive Aromatisierung. Andererseits wird der im festen Trägerstoff gebundene mindestens eine Geschmacks- und/oder Geruchsstoff während eines langen Zeitraumes sehr gleichmäßig freigesetzt. Die in dieser Weise aromatisierten zellulosehaltigen Produkte 1 können somit über Wochen und sogar Monate gelagert werden, ohne dass die Intensität der Aromatisierung spürbar nachlässt.

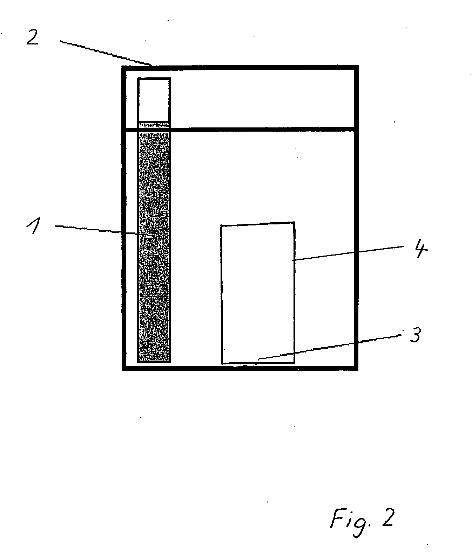

Eine weitere Form der erfindungsgemäßen Aromatisierung von zellulosehaltigen Produkten ist rein schematisch in Figur 2 dargestellt. In dem in Figur 2 dargestellten Ausführungsbeispiel handelt es sich bei den zellulosehaltigen Produkten 1 wiederum um Filterzigaretten, die in Hartschachteln mit gasdichter Zellophanhülle verpackt sind. Das Einbringen von dem mindestens einen in einen festen Trägerstoff gebundenen Geschmacks- und/oder Geruchsstoff 3 in die Verpackung 2 für die zellulosehaltigen Produkte 1 geschieht hier, indem der feste Trägerstoff mit dem darin gebundenen mindestens einem Geschmacks- und/oder Geruchsstoff 3 auf ein Stützmaterial 4 auf- oder in dieses Stützmaterial 4 eingebracht wird und das in dieser Weise präparierte Stützmaterial 4 in die Verpackung 2 eingelegt wird. Als Stützmaterial 4 ist ein Flachmaterial, wie z. B. Papier, Karton sowie Kunststoff- oder Metallfolie besonders geeignet. Der feste Trägerstoff mit dem darin gebundenen mindestens einem Geschmacks- und/oder Geruchsstoff 3 kann z. B. im flüssigen Zustand auf das Stützmaterial 4 aufgesprüht oder aufgegossen werden. Es ist auch möglich, das Stützmaterial 4 zu beschichten, indem das Stützmaterial 4 in eine Schmelze des Trägerstoffes und des darin gebundenen mindestens einen Geschmacks- und/oder Geruchsstoffes 3 eingetaucht wird und der sich nach der Entnahme des Stützmaterials 4 aus der Schmelze anhaftende Trägerstoff mit dem gebundenen Geschmacks- und/oder Geruchsstoff bei Raumtemperatur oder durch zusätzliche Kühlung zu einer Schicht verfestigt.

Poröse Stützmaterialien 4, wie saugfähiges Papier, Gewebe, Vlies usw. können auch getränkt werden, indem sie in eine Schmelze des Trägerstoffes mit dem darin gebundenen mindestens einem Geschmacks- und/oder Geruchsstoff 3 eingetaucht werden. Nach der Entnahme aus der Schmelze verfestigt sich auch hier der in das Stützmaterial 4 eingedrungene sowie der an der Oberfläche anhaftende Trägerstoff mit dem eingelagerten Geschmacks- und/oder Geruchsstoff 3 durch Abkühlung und verbindet sich dadurch fest mit dem Stützmaterial 4. Weitere Stützmaterialien können Keramik oder Glas sein, sowie gewebeartige Produkte aus Stoff, Glas, Keramik, Metall oder Kunststofffasern.

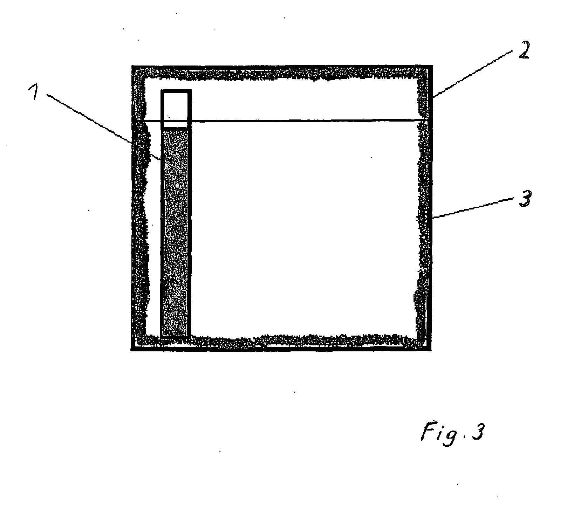

In Figur 3 ist rein schematisch eine weitere Ausführungsform des erfindungsgemäβen Verfahrens zur Aromatisierung von zellulosehaltigen Produkten 1 dargestellt, bei welcher als Stützmaterial 4 für die Auf- oder Einbringung des Trägerstoffes mit dem darin gebundenen mindestens einem Geschmacks- und/oder Geruchsstoff 3 die Innenseite des Verpackungsmaterials 2 selbst dient. Zur Beschichtung der Innenseite des Verpackungsmaterials 2 eignet sich besonders das Besprühen aus einer Schmelze des Trägerstoffes mit dem darin gebundenen mindestens einem Geschmacks- und/oder Geruchsstoff 3 und die anschließende Verfestigung durch Abkühlung.

Selbstverständlich kann dieses Verpackungsmaterial auch zum Schutz empfindlicher Produkte 1 vor, wie in Fig. 1 gezeigten, granulatartigen Trägerstoffe verwendet werden. Oder die in Fig. 3 mit dem Trägermaterial beschichteten Innenseiten der Produktverpackung 2 können mit einer zusätzlichen Schicht aus einem solchen Verpackungsmaterial versehen werden. Wichtig ist nur, dass dieses Verpackungsmaterial die Aromastoffe 3 hindurchtreten lässt.

Das Stützmaterial 4, 14, auf dem der Trägerstoff mit den gebundenen Aromastoffen (3) aufgebracht ist, kann in der Verpackung 2 sowohl außerhalb als auch innerhalb des Produktes 1 angeordnet sein.

Es sei die Anwendungsmöglichkeit eines verpackten Duftstoffträgers in einem PE-Beutel bei der Beduftung von Handtüchern genannt, die in einer Mikrowelle beispielsweise eines Flugzeugs erwärmt werden. So wurden in einem Test Baumwolltücher (nicht gezeigt) kurze Zeit erwärmt, die in einer Verpackung zusammen mit einem PE-Beutel angeordnet waren, in dem ein in einem Trägerstoff eingeschlossener und auf einem Stützmaterial angeordneter Duftstoff eingeschlossen war. Als Handtücher kamen übliche Gästetücher zum Einsatz, zwischen die ein in einem PE-Beutel angeordnetes kartenartiges Stützmaterial gelegt war, wobei auf dem Stützmaterial ein Dünnfilm aus Trägerstoff und Duftstoff aufgebracht war. Die Mikrowelle wurde dabei mit 800 W für 30 s betrieben. Nach Entnahme der Tücher aus der Mikrowelle war ein wesentlich stärkerer Geruchseindruck auf den Tüchern wahrzunehmen als in einem Vergleichsversuch über 30 s, bei dem keine Mikrowellenbestrahlung zum Einsatz kam. Damit ist gezeigt, dass die Eintragung von Energie eine sehr deutliche Beschleunigung des Aromatisierungsvorganges bewirken kann.

Die in dem Versuch verwendeten Tücher waren trocken. Wenn sie nass verwendet werden, ist eine zusätzliche Beschleunigung zu erwarten, da dann die Mikrowellenstrahlung stärker absorbiert werden kann. Wesentlich ist somit, dass die eingestrahlte Energieform entweder durch die Waren selbst oder durch das Stützmaterial oder den Trägerstoff bzw. den Duftstoff selbst absorbiert werden kann, um so die Freisetzung des Duftstoffes aus dem Trägerstoff zu beschleunigen.

Die Aromatisierung kann dabei auf verschiedene Weise erfolgen. Die Trägerstoffe können gesondert verpackt sein, zum Schutz der Produkte. Diese Verpackung kann jedoch auch weggelassen werden, wenn die Produkte gegenüber den Trägerstoffen unempfindlich sind. Die Produkte können in einer Verpackung angeordnet sein. Die Verpackung der Produkte kann jedoch auch weggelassen werden. Beispielsweise ist dies in einer Mikrowelle nicht notwendig, da hier das Mirkowellengehäuse die Funktion der Verpackung übernimmt. Dabei können die Trägerstoffe mit den gebundenen Geruchs- oder Geschmacksstoffen an, in oder in der Nähe der Produkte angeordnet sein.

Vorteilhaft ist eine äußere Versiegelung (nicht gezeigt) der Produktverpackung 2, da damit zum einen eine Verminderung der Substanzkonzentration im Inneren der Verpackung 2 und zum anderen auch eine ungewollte Beeinflussung weiterer benachbart gelagerter Produkte (nicht gezeigt) verhindert wird. Als Material für die Versiegelung kommen insbesondere Zellophan oder eine Metallfolie in Betracht, die auch gleich einstückig auf der Produktverpackung 2 angeordnet sein können.

Das erfindungsgemäße Verfahren zur Aromatisierung, bei welchem die zellulosehaltigen Produkte 1 nach deren Verpackung innerhalb des Verpackungsmaterials 2 indirekt aromatisiert werden, hat gegenüber der bekannten direkten Aromatisierung der zellulosehaltigen Produkte 1 während des Produktionsprozesses folgende wesentlichen Vorteile:
a) Der Produktionsprozess für die Produkte 1 bleibt von der Aromatisierung unbeeinflusst.
b) Die Aromatisierung beginnt erst mit der Zuordnung der Aromastoffe zu den mit den zellulosehaltigen Produkten 1 bzw. der Verpackung der zellulosehaltigen Produkte 1 und somit später als bereits bei deren Produktion, d. h. in gröβerer zeitlicher Nähe zum Verbrauch.
c) Durch die Bindung des mindestens einen Geschmacks- und/oder Geruchsstoffes 3 an einen bei Normaltemperatur festen Trägerstoff erfolgt dessen Freisetzung und Übertragung auf die in der Verpackung befindlichen Produkte 1 über einen langen Zeitraum, wobei der hohe Anteil des Geschmacks- und/oder Geruchsstoffs 3 von bis zu 60 Gew.-% gleichzeitig eine intensive Aromatisierung gewährleistet.
d) Die zu aromatisierenden Produkte 1 können saisonal unterschiedlich aromatisiert werden, z. B. mit erfrischenden Sommerdüften oder stimmungserregenden Weihnachtsdüften. Eine solche saisonale Umstellung ist jederzeit und ohne größere Vorbereitungen möglich; es muss lediglich für einen Wechsel des in die Verpackungen eingegebenen mindestens einen Geschmacks- und/oder Geruchsstoffes 3, welcher in einem bei Normaltemperatur festen Trägerstoff gebunden ist, gesorgt werden.

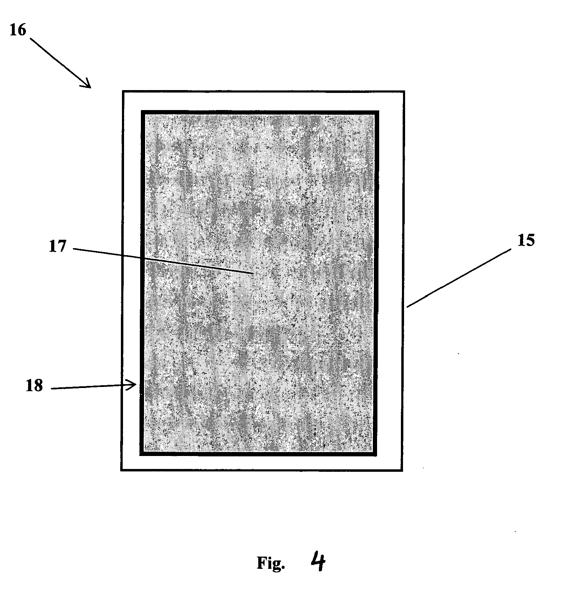

In Fig. 4 ist ein erfindungsgemäßes Mittel zur Aromatisierung rein schematisch dargestellt. Und zwar zeigt Fig. 4 ein Mittel 16, das aus einem Stützmaterial besteht, das mit einem Trägerstoff 17 versehen ist, in dem ein Geruchsstoff aufgenommen ist, und eine Filmform 18 bildet. Die Filmform 18 ist mit einer Umhüllung 15 gegenüber der Umgebung versiegelt, so dass weder der Trägerstoff 17 noch das Stützmaterial nach außen dringen können oder die Umgebung mit diesen Materialien in Kontakt treten kann. Die Umhüllung 15 besteht aus PE und ist für den Geruchsstoff durchlässig.

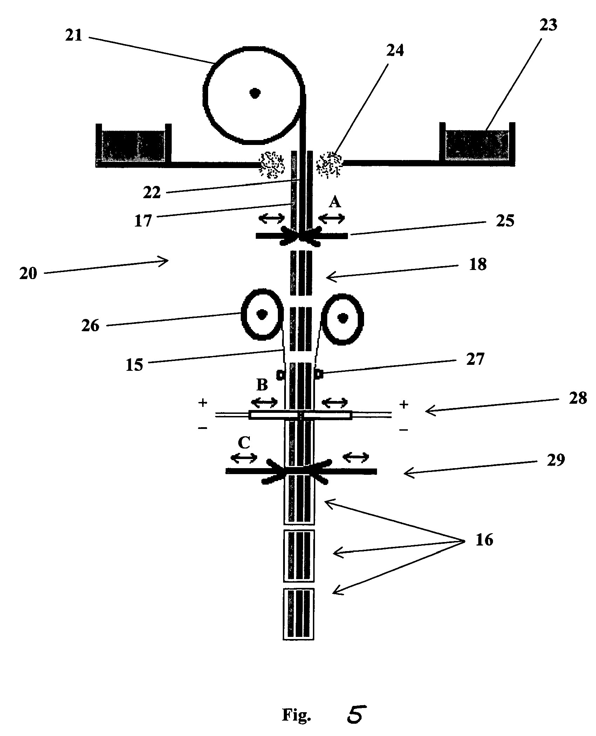

Die zur Herstellung dieses erfindungsgemäßen Mittels 16 verwendete Vorrichtung 20 ist in Fig. 5 rein schematisch dargestellt. Von einer Papierrolle 21 wird Papier 22 abgewickelt und dadurch mit einer Beschichtung aus dem Trägerstoff 17 versehen, dass eine Schmelze 23 aus dem Trägerstoff 17 und dem darin gelösten Geruchsstoff als Spray 24 auf dem Papier 22 aufgesprüht wird. Zur Herstellung der Schmelze können beispielsweise Granulate aufgeschmolzen werden. Alternativ kann die Schmelze 23 auch aufgegossen werden. Nach dem Abkühlen der aufgesprühten Schmelze 17 wird das beschichtete Endlospapier 22 in bedarfsgerechte Stücke mit Hilfe einer Schneidvorrichtung 25 zertrennt, bei der Schneidmesser in der senkrecht zum Papier 22 angeordneten Schnittrichtung A geführt werden, wodurch Filmformen 18 gebildet werden.

Die Filmformen 18 werden nachfolgend dadurch umhüllt, dass eine PE-Folie 15, die auf beidseits der Stückführung angeordneten Vorratsrollen 26 aufgenommen ist, über Andrückrollen 27 an die Filmformen 18 angelegt und jede einzelne Filmform 18 über eine Heizeinrichtung 28 mit der PE-Folie 15 versiegelt wird. Beim Versiegeln werden die beiden Folienseiten 15 jeweils durch Aufeinanderdrücken von Heizstempeln der Heizeinrichtung 28 in einer Richtung senkrecht zur Stückführung über Hitze miteinander verbunden. So wird jede einzelne Filmform 18 entlang ihrer Außenkontur versiegelt.

Die dadurch zwischen einzelnen Filmformen 18 entstehenden Stege aus PE-Folie werden anschließend durch eine weitere Schneideinrichtung 29, bei der Schneidmesser wiederum in einer zur Stückführung senkrechten Richtung C geführt werden, aufgetrennt, so dass die erfindungsgemäßen Mittel 16 entstehen, die aus einer Umhüllung 15 bestehen, in der das mit der Beschichtung 17 versehene Papier 22, also eine Filmform 18, aufgenommen und versiegelt ist.

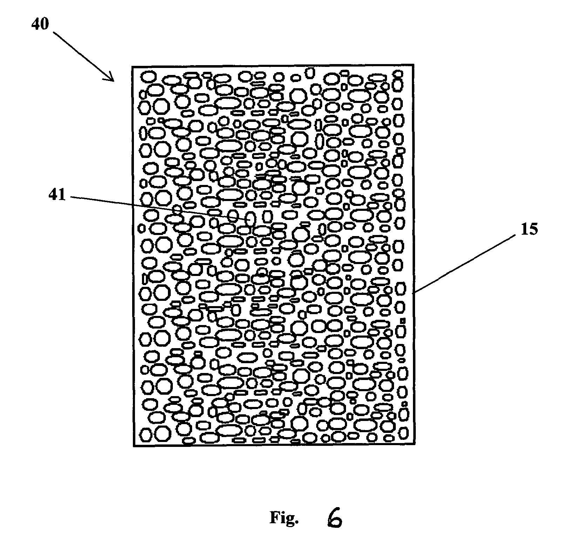

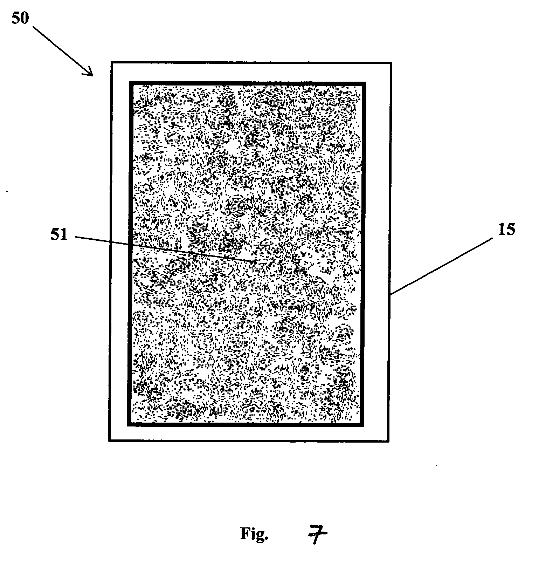

In Fig. 6 und 7 sind alternative Mittel 40 bzw. 50 dargestellt, die sich von dem Mittel 16 dadurch unterscheiden, dass beim Mittel 40 in die Umhüllung 15 ein Granulat, beispielsweise Chips 41, und beim Mittel 50 ein Pulver, also ein Puder 51, versiegelt ist.

Durch die erfindungsgemäßen Mittel 16, 40, 50, also eine Kombination von Filmform 18, Granulat 41 oder Pulver 51 mit einer bestimmten Umhüllung 15, können somit Nachfüllpackungen bereitgestellt werden, die auf die unterschiedlichsten Anwendungsfälle gezielt einstellbar sind. Hauptanwendungsfälle sind dabei die Zuführung von Geruchs- oder Geschmacksstoffen auf bzw. in andere Stoffe, wie zellulosehaltige Stoffe.

Für Raumtemperaturanwendungen wird eine Umhüllung 15 aus LDPE bevorzugt, für Temperaturen bis zu 80 °C eine Umhüllung 15 aus HDPE und für Temperaturen im Bereich von 80 °C bis zu 200 °C eine Umhüllung 15 aus Silikon. Folien 15 von etwa 0,5 mm Dicke sind dabei bevorzugt, da sie eine ausreichende Durchlässigkeit bei gleichzeitig akzeptabler Festigkeit aufweisen. Für besondere Anwendungen kann die freigesetzte Menge an Geruchs- oder Geschmacksstoffen gezielt über die Umhüllungsdicke und/oder die Wahl der Darreichungsform der in einem Trägerstoff aufgenommenen Geruchs- oder Geschmacksstoffe und/oder die Verwendung von bestimmten Lösungsmitteln eingestellt werden. Dabei können die Freisetzungsraten noch zusätzlich erhöht werden durch die Einbringung von Energie.

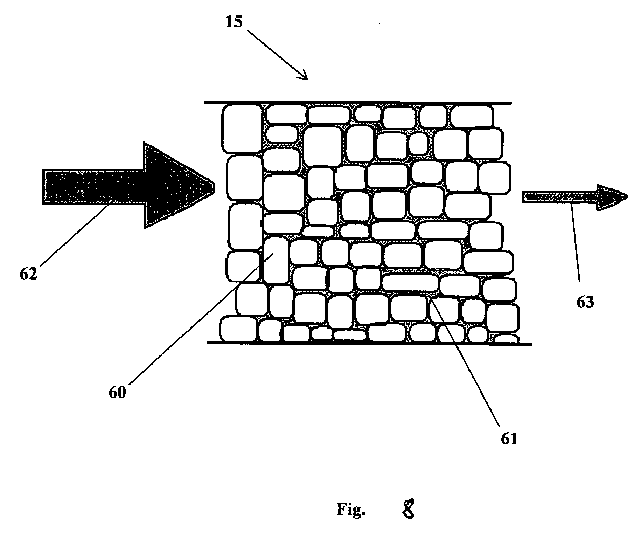

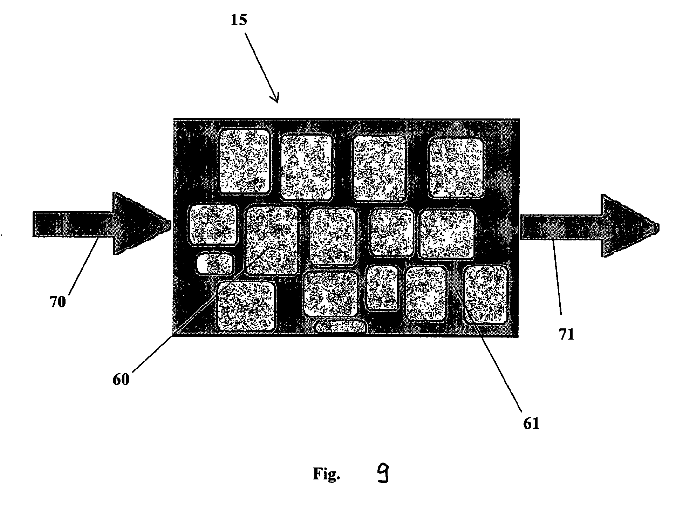

Um die Wirkung eine Lösungsmittels zu verdeutlichen, sind in den Fig. 8 und 9 die grundsätzlichen Unterschiede bei einem Durchtritt von polaren Substanzen durch eine PE-Folie 15 dargestellt. In Fig. 8 ist zu erkennen, dass die PE-Folie 15 aus einzelnen Polyolefinpartikeln 60 besteht, zwischen denen Zwischenräume 61 angeordnet sind. Eine solche PE-Folie 15 ist nicht polar. Eine sich auf einer Seite der PE-Folie 15 befindliche polare Substanz 62, beispielsweise ein Geruchsstoff, wird durch seine Polarität am Durchtritt der Folie 15 stark behindert und nur die Zwischenräume 61 gestatten den Durchtritt einer geringen Menge der Substanz 63.

In Fig. 9 ist rein schematisch die Auswirkung aufgezeigt, wenn vorgesehen ist, die polare Substanz mit einem nicht polaren Lösungsmittel zu einer Substanz 70 zu vermengen. Die PE-Folie 15 mit den Polyolefinpartikeln 60 nimmt die Substanz 70 auf, da das nicht polare Lösungsmittel in den Polyolefinpartikeln 60 gelöst wird. Dadurch quellen die Polyolefinpartikel 60 auf, es vergrößern sich die Zwischenräume 61 und die Folie 15 quillt auf, was ein aus der Literatur durchaus bekannter Effekt ist. Nun kann die Substanz 70 im Prinzip ungehindert durch die Folie 15 hindurchtreten, wobei die durchgelassene Menge 71 wesentlich größer ist, als bei Fig. 8.

Es ist ersichtlich geworden, welche bedeutenden Vorteile die vorliegende Erfindung bereitstellt. Dabei ist eine Aromatisierung auf zellulosehaltige Produkte, die Tabak aufweisen, möglich.

## Patentansprüche

1. Verfahren zur Aromatisierung von zellulosehaltigen Produkten (1), wobei das Produkt (1) Tabak aufweist, umfassend
Anordnen von mindestens einem in einem festen Trägerstoff gebundenen Geschmacks- und/oder Geruchsstoff (3) in Bezug auf das Produkt auf zumindest eine der Arten: an dem Produkt (1) anordnen oder so nah in unmittelbarer Nähe des Produkts (1) anordnen, dass eine ausreichende Menge Geschmacks- oder Geruchsstoff zu dem Produkt gelangen kann,
**dadurch gekennzeichnet, dass**
der bei Normaltemperatur flüssige Geschmacks- oder Geruchsstoff in dem bei Normaltemperatur festen Trägerstoff eingekapselt ist, so dass der feste Trägerstoff die Geschmacks - oder Geruchsstoffe nicht nur speichert und Konserviert, sondern über die Lagerzeit Kontrolliert wieder abgibt.

2. Verfahren nach Anspruch 1,
**dadurch gekennzeichnet,**
**dass** das Produkt (1) in einer Verpackung (2) angeordnet ist, in der auch der feste Trägerstoff angeordnet ist und/oder der feste Trägerstoff mit dem darin gebundenen mindestens einem Geschmacks- bzw. Geruchsstoff (3) in Form von Pulver (51), Granulat, Chips (41), Perlen oder dergleichen verwendet wird.

3. Verfahren nach Anspruch 1 oder 2,
**dadurch gekennzeichnet,**
**dass** der feste Trägerstoff mit dem darin gebundenen mindestens einem Geschmacks- bzw. Geruchsstoff (3) auf ein Stützmaterial (4) auf- oder in das Stützmaterial (4eingebracht wird, wobei bevorzugt das Stützmaterial (4) ein Flachmaterial oder ein durch Ur- oder Umformen erzeugtes oder aus mehreren Elementen zusammengesetztes Teil beliebiger Form ist, vorzugsweise Papier, Karton, Kunststoff- oder Metallfolie, Keramik, Glas, Vlies oder Gewebe oder ein gewebeartiges Produkt aus Stoff, Glas, Keramik, Metall oder Kunststofffasern.

4. Verfahren nach Anspruch 3,
**dadurch gekennzeichnet,**
**dass** der feste Trägerstoff mit dem darin gebundenen mindestens einem Geschmacks- bzw. Geruchsstoff (3) aus einer Schmelze des Trägerstoffes und des darin gebundenen mindestens einem Geschmacks- bzw. Geruchsstoffes (3) auf das Stützmaterial (4) auf- oder in das Stützmaterial (4) eingebracht und anschließend durch Abkühlen verfestigt wird, wobei bevorzugt das Stützmaterial (4) aus einer Schmelze des Trägerstoffes mit dem darin gebundenen mindestens einem Geschmacks- bzw. Geruchsstoffes (3) beschichtet wird, wobei das Stützmaterial (4) insbesondere mit der Schmelze besprüht, begossen oder getränkt wird.

5. Verfahren nach Anspruch 1 und/oder einem der Ansprüche 3 oder 4,
**dadurch gekennzeichnet,**
**dass** als Stützmaterial (4) für die Auf- oder Einbringung des Trägerstoffes mit dem darin gebundenen mindestens einem Geschmacks- bzw. Geruchsstoff (3) die Innenseite des Verpackungsmaterials (2) selbst dient.

6. Verfahren nach einem der vorherigen Ansprüche bzw. einem der Ansprüche 3 bis 5,
**dadurch gekennzeichnet,**
**dass** der Trägerstoff gegenüber dem Produkt (11) durch eine für den Trägerstoff bzw. den Trägerstoff und das Stützmaterial undurchdringbare Umhüllung (15) versiegelt wird, die für die Aromatisierungsstoffe zumindest in einem Bereich der Umhüllung (15) durchlässig ist, wobei bevorzugt die Umhüllung (15) in diesem Bereich aus Papier, Polyethylen, insbesondere modifiziertes Polyethylen, Low Density Polyethylen oder High Density Polyethylen, oder Silikonfolie besteht.

7. Verfahren nach Anspruch 6,
**dadurch gekennzeichnet,**
**dass** der Durchtritt der Aromatisierungsstoffe durch die Umhüllung eingestellt wird durch die Zugabe eines polaren oder nichtpolaren Lösungsmittels für den Geschmacks- bzw. Geruchsstoff.

8. Verfahren nach einem der vorherigen Ansprüche,
**dadurch gekennzeichnet,**
**dass** die Verpackung (2) ein für den Geschmacks- bzw. Geruchsstoff (3) undurchlässiges Material, wie Zellophan, umfasst, das insbesondere einstückig mit der Verpackung (2) verbunden ist.

9. Verfahren nach einem der vorherigen Ansprüche,
**dadurch gekennzeichnet,**
**dass** die Aromatisierung von dem Produkt (1) **dadurch** beschleunigt wird, dass der Trägerstoff und/oder das Produkt (1) und/oder die Verpackung (2) mit Energie beaufschlagt, insbesondere mit elektromagnetischer Strahlung oder Ultraschall bestrahlt wird, wobei bevorzugt als elektromagnetische Strahlung Mikrowellen- und/oder Wärmestrahlung eingesetzt wird.

10. System aus wenigstens einem zellulosehaltigen Produkt (1), wobei das Produkt (1) Tabak aufweist, und
wenigstens einem Mittel (16) zur Aromatisierung von Produkten, insbesondere zellulosehaltigen Produkten nach dem Verfahren nach einem der Ansprüche 1 bis 9, mit mindestens einem in einem festen Trägerstoff (17) gebundenen Geschmacks- und/oder Geruchsstoff (3), wobei der Trägerstoff (17) bevorzugt in einer Umhüllung (15) eingeschlossen ist, die den Durchtritt des Trägerstoffs (17) verhindert und zumindest in einem Bereich der Umhüllung (15) für den Geruchs- bzw. Geschmacksstoff (3) durchlässig ist,
**dadurch gekennzeichnet, dass**
der bei Normaltemperatur flüssige Geschmacks- oder Geruchsstoff in dem bei Normaltemperatur festen Trägerstoff eingekapselt ist, so dass der feste Trägerstoff die Geschmacks- oder Geruchsstoffe nicht nur speichert und Konserviert, sondern über die Lagerzeit Kontrolliert wieder abgibt und wobei der mindestens eine in dem festen Trägerstoff gebundene Geschmacks- und/oder Geruchsstoff (3) in Bezug auf das Produkt auf zumindest eine der Arten angeordnet ist: an dem Produkt (1) oder so nah in unmittelbarer Nähe des Produkts (1), dass eine ausreichende Menge Geschmacks- oder Geruchsstoff zu dem Produkt gelangen kann.

11. System nach Anspruch 10,
**dadurch gekennzeichnet**
**dass** die Umhüllung Papier, Polyethylen (15), insbesondere modifiziertes Polyethylen, Low Density Polyethylen oder High Density Polyethylen, oder Silikonfolie umfasst und wobei insbesondere dem Geschmacks- bzw. Geruchsstoff (3) ein polares oder nichtpolares Lösungsmittel beigegeben ist zur Einstellung des Durchtritts der Aromatisierungsstoffe durch die Umhüllung.

12. System nach Anspruch 10 oder 11,
**dadurch gekennzeichnet**
**dass** der feste Trägerstoff mit dem darin gebundenen mindestens einem Geschmacks- bzw. Geruchsstoff (3) in Form von Pulver (51), Granulat, Chips (41), Perlen oder dergleichen vorliegt, insbesondere in Form von Pulver (51) mit einem Außendurchmesser im Bereich von 10 µm bis 100 µm, bevorzugt 25 µm bis 50 µm, oder in Form von Granulat (41), Chips oder Perlen mit einem Außendurchmesser von bis zu 10 mm, vorzugsweise bis zu 5 mm.

13. System nach Anspruch 10 bis 12,
**dadurch gekennzeichnet,**
**dass** der feste Trägerstoff (17) mit dem darin gebundenen mindestens einem Geschmacks- bzw. Geruchsstoff (3) auf einem Stützmaterial auf oder in das Stützmaterial eingebracht ist, wobei das Stützmaterial bevorzugt ein Flachmaterial oder ein durch Ur- oder Umformen erzeugtes oder aus mehreren Elementen zusammengesetztes Teil beliebiger Form ist, insbesondere Papier (22), Karton, Kunststoff- oder Metallfolie, Keramik, Glas, Vlies oder Gewebe sowie ein gewebeartiges Produkt aus Stoff, Glas, Keramik, Metall oder Kunststofffasern, wobei die Umhüllung insbesondere für das Stützmaterial undurchlässig ist, wobei bevorzugt das Stützmaterial für die Auf- oder Einbringung des Trägerstoffes mit dem darin gebundenen mindestens einem Geschmacks- bzw. Geruchsstoff die Innenseite des Materials einer Verpackung des Produktes ist.

14. System nach einem der Ansprüche 10 bis 13,
**dadurch gekennzeichnet,**
**dass** die Umhüllung (15) eine Dicke von 0,1 mm bis 2 mm, bevorzugt 0,2 mm bis 0,5 mm aufweist und/oder der mindestens eine Geschmacks- bzw. Geruchsstoff (3) in einem nicht polaren Lösungsmittel, bevorzugt einem iso-paraffinisches Lösungsmittel, gelöst ist, das vorzugsweise einen Flammpunkt von größer als 50 °C, insbesondere größer als 60 °C aufweist, wobei die auf die gesamte Menge von Lösungsmittel und Geschmacks- bzw. Geruchsstoff (3) bezogene Menge des Lösungsmittels 0,5% bis 50%, insbesondere 10% beträgt.

15. System nach einem der Ansprüche 10 bis 14,
**dadurch gekennzeichnet,**
**dass** das System weiterhin eine Verpackung (12) umfasst, die die Mittel und das Produkt umschließt, wobei die Verpackung (12) insbesondere ein für den Geschmacks- bzw. Geruchsstoff (3) undurchlässiges Material, wie Zellophan, umfasst, das bevorzugt einstückig mit der Verpackung (12) verbunden ist.

## Claims

1. A process for the flavouring of cellulose-containing products (1), the product comprising tobacco, comprising:
at least one flavour and/or fragrance (3) bound in a solid carrier material is arranged with respect to the product in at least one of the following ways: arranging at the product (1) or in such an immediate vicinity of the product (1) that an adequate amount of flavour or fragrance can be transported to the product,
**characterized in that**
the flavour or fragrance being liquid at normal temperature is encapsulated by the carrier material being solid at normal temperature, so that the solid carrier material not only stores and preserves the flavour or fragrance but also delivers it gradually in a controlled manner.

2. Process in accordance with claim 1,
**characterized in that**
the product (1) is contained in a packaging (2) which also contains the solid carrier material and/or the solid carrier material with the at least one flavour or fragrance (3) bound therein is used in the form of powder (51), granules, chips (41), pearls or the like.

3. Process in accordance with claim 1 or 2,
**characterized in that**
the solid carrier material with the at least one flavour or fragrance (3) bound therein is applied onto a support material (4) or is inserted into the support material (4), wherein preferentially the support material (4) is a flat material or a part of any shape created by basic forming or transforming or combined from several elements, preferably paper, cardboard, plastic sheeting or metal foil, ceramics, glass, non-woven material or tissue or a tissue-like product of textile material, glass, ceramics, metal or plastics fibres.

4. A process in accordance with claims 3,
**characterized in that**
the solid carrier material with the at least one flavour or fragrance (3) bound therein is applied to the support material (4) or is inserted into the support material (4) from a melt of the carrier material and the at least one flavour or fragrance (3) bound therein, and then solidified by cooling, wherein preferentially the support material (4) is coated from a melt of the carrier material with the at least one flavour or fragrance (3), wherein the support material (4) is in particular sprayed, poured or soaked with the melt.

5. Process in accordance with claim 1 and/or any of the claims 3 or 4,
**characterized in that**
the inner side of the packaging material (2) itself is used as support material (4) for the application or insertion of the carrier material with the at least one flavour or fragrance (3) bound therein.

6. Process in accordance with any of the preceding claims or any of claims 3 to 5, **characterized in that**
the carrier material is sealed off in relation to the product (11) by means of an enclosure (15) which is impermeable to the carrier material or to the carrier material and the support material, but is permeable to the flavouring in at least one area of the enclosure (15), wherein preferentially the enclosure (15) in this area consists of paper, polyethylene, in particular modified polyethylene, low density polyethylene or high density polyethylene or silicone sheet.

7. Process in accordance with claim 6,
**characterized in that**
passage of the flavouring through the enclosure is adjusted by means of addition of a polar or non-polar solvent for the flavour or fragrance.

8. Process in accordance with any of the preceding claims,
**characterized in that**
the packaging (2) comprises a material impermeable to the flavour or fragrance (3), such as cellophane, which is in particular connected as one piece with the packaging (2).

9. Process in accordance with any of the preceding claims,
**characterized in that**
the flavouring of the product (1) is accelerated by exposing the carrier material and/or the product (1) and/or the packaging (2) to an energy source, especially to electromagnetic radiation or ultrasound, wherein preferentially the electromagnetic radiation applied is microwave and/or thermal radiation.

10. System from at least one cellulose-containing products (1), the product containing tobacco, and at least one means (16) for the flavouring of products, in particular a cellulose-containing product (11) in accordance with any of the Claims 1 to 9, the means having at least one flavour and/or fragrance (3) bound in a solid carrier material (17), wherein preferentially the carrier material (17) is enclosed in an enclosure (15) that prevents passage of the carrier material (17) and is permeable to the fragrance or flavour in at least one area of the enclosure,
**characterized in that**
the flavour or fragrance being liquid at normal temperature is encapsulated by the carrier material being solid at normal temperature, so that the solid carrier material not only stores and preserves the flavour or fragrance but also delivers it gradually in a controlled manner and wherein the at least one flavour and/or fragrance (3) bound in a solid carrier material is arranged with respect to the product in at least one of the following ways: arranging at the product (1) or in such an immediate vicinity of the product (1) that an adequate amount of flavour or fragrance can be transported to the product.

11. System in accordance with Claim 10,
**characterized in that**
the enclosure comprises paper, polyethylene (15), in particular modified polyethylene, low density polyethylene or high density polyethylene, or silicone sheet and wherein in particular a polar or non-polar solvent is added to the flavour or fragrance (3) for adjusting the passage of the flavouring through the enclosure.

12. System in accordance with Claim 10 or 11,
**characterized in that**
the solid carrier material with the at least one flavour or fragrance (3) bound therein is present in the form of powder (51), granules, chips (41), pearls or the like, in particular in the form of powder (51) with an external diameter in the range of 10 µm to 100 µm, preferably 25 µm to 50 µm, or in the form of granulate (41), chips or pearls with an outer diameter of up to 10 mm, preferably up to 5 mm.

13. System in accordance with claims 10 to 12,
**characterized in that**
the solid carrier material (17) with the at least one flavour or fragrance (3) bound therein is applied to or inserted into the support material, wherein the support material is preferably a flat material or a part of any shape created by basic forming or transforming or combined from several elements, in particular paper (22), cardboard, plastic sheeting or metal foil, ceramics, glass, non-woven material or tissue as well as a tissue-like product from textile material, glass, ceramics, metal or plastics fibres, wherein the enclosure is in particular impermeable to the support material, wherein preferentially the support material for the application or insertion of the carrier material with at least one flavour or fragrance bound therein, constitutes the inner side of the material of a packaging of the product.

14. System in accordance with any of Claims 10 to 13,
**characterized in that**
the enclosure (15) has a thickness of 0,1 mm to 2 mm, preferably 0,2 mm to 0,5 mm and/or the at least one flavour or fragrance (3) is dissolved in a non-polar solvent, preferably an isoparaffinic solvent, which preferably has a flash point of greater than 50 °C, in particular greater than 60 °C, wherein the volume of solvent related to the entire amount of solvent plus flavour or fragrance (3) amounts to 0,5% to 50%, in particular 10%.

15. System in accordance with any of Claims 10 to 14,
**characterized in that**
the system furthermore comprises a packaging (12) that encloses the means (16) and the product (11), wherein the packaging (12) in particular comprises a material impermeable to the fragrance or flavour (3), such as cellophane, which is preferably connected as one piece with the packaging (12).

## Revendications

1. Procédé d'aromatisation de produits (1) contenant de la cellulose, le produit (1) présentant du tabac, comprenant
la disposition d'au moins une substance de sapidité et/ou odorante (3), liée dans une substance formant support solide d'au moins une des façons par rapport au produit : disposition sur le produit (1) ou bien suffisamment près à proximité immédiate du produit (1) pour qu'une quantité suffisante de substance de sapidité et/ou odorante puisse parvenir au produit,
**caractérisé en ce que**
la substance de sapidité et/ou odorante, liquide à la température normale, est encapsulée dans la substance formant support solide à la température normale, de sorte que la substance formant support solide non seulement emmagasine et conserve les substances de sapidité et/ou odorantes mais les restitue de façon contrôlée pendant la durée de conservation.

2. Procédé selon la revendication 1,
**caractérisé en ce que**
le produit (1) est disposé dans un emballage (2) dans lequel la substance formant support solide est également disposée et/ou la substance formant support solide est utilisée avec au moins une substance de sapidité et/ou odorante (3) qui lui est liée sous la forme de poudre (51), de granulés, de paillettes (41), de perles ou similaires.

3. Procédé selon la revendication 1 ou la revendication 2,
**caractérisé en ce que**
la substance formant support solide avec au moins une substance de sapidité et/ou odorante (3) qui lui est liée est mise en place sur un matériau d'appui (4) ou dans le matériau d'appui (4), le matériau d'appui (4) étant de préférence un matériau plat ou une partie produite par formage initial ou déformation ou une partie de forme quelconque assemblée à partir de plusieurs éléments, de préférence du papier, du carton, une feuille en matière plastique ou en métal, de la céramique, du verre, du non-tissé ou un tissu ou un produit de type tissu en étoffe, en verre, en céramique en métal ou en fibres de matière plastique.

4. Procédé selon la revendication 3,
**caractérisé en ce que**
la substance formant support solide avec au moins une substance de sapidité et/ou odorante (3) qui lui est liée est mise en place sur le matériau d'appui (4) ou dans le matériau d'appui (4) à partir d'une coulée de la substance formant support et d'au moins une substance de sapidité et/ou odorante (3) qui lui est liée et puis est solidifiée par refroidissement, le matériau d'appui (4) étant de préférence appliqué en revêtement à partir d'une coulée de la substance formant support avec au moins une substance de sapidité et/ou odorante (3) qui lui est liée, le matériau d'appui (4) recevant en particulier la coulée par pulvérisation, versement ou humectation.

5. Procédé selon la revendication 1 ou selon l'une des revendications 3 ou 4,
**caractérisé en ce que**
le côté intérieur du matériau d'emballage (2) lui-même sert de matériau d'appui (4) pour l'application en surface ou intérieure de la substance formant support avec au moins une substance de sapidité et/ou odorante (3) qui lui est liée.

6. Procédé selon l'une des revendications précédentes ou selon l'une des revendications 3 à 5
**caractérisé en ce que**
la substance formant support est scellée par rapport au produit (11) par une enveloppe (15) impénétrable par la substance formant support ou par la substance formant support et le matériau d'appui, enveloppe qui est perméable aux substances d'aromatisation au moins dans une zone de l'enveloppe (15), l'enveloppe (15) se composant de préférence dans cette zone de papier, de poly(éthylène), en particulier de poly(éthylène) modifié, de poly(éthylène) basse densité ou de poly(éthylène) haute densité, ou de feuille de silicone.

7. Procédé selon la revendication 6,
**caractérisé en ce que**
le passage des substances d'aromatisation à travers l'enveloppe est assuré par l'addition d'un solvant polaire ou non polaire pour la substance de sapidité et/ou odorante.

8. Procédé selon l'une des revendications précédentes,
**caractérisé en ce que**
l'emballage (2) comprend un matériau imperméable à la substance de sapidité et/ou odorante, comme de la cellophane, qui est raccordé de préférence d'un seul tenant à l'emballage (2).

9. Procédé selon l'une des revendications précédentes,
**caractérisé en ce que**
l'aromatisation du produit (1) est accélérée par le fait que la substance formant support et/ou le produit (1) et/ou l'emballage (2) est alimenté(e) en énergie, est en particulier exposé à un rayonnement électromagnétique ou à des ultrasons, un rayonnement par micro-ondes et/ou un rayonnement thermique étant en particulier utilisé en tant que rayonnement électromagnétique.

10. Système constitué d'au moins un produit (1) contenant de la cellulose, le produit (1) présentant du tabac, et au moins un moyen (16) pour l'aromatisation de produits, en particulier de produits contenant de la cellulose, selon un procédé selon l'une des revendications 1 à 9, avec au moins une substance de sapidité et/ou odorante (3) liée dans une substance formant support (17) solide, la substance formant support (17) étant de préférence incluse dans une enveloppe (15) qui empêche la pénétration de la substance formant support (17) et qui est perméable à la substance de sapidité et/ou odorante (3) au moins dans une zone de l'enveloppe (15), **caractérisé en ce que**
la substance de sapidité et/ou odorante, liquide à la température normale, est encapsulée dans la substance formant support solide à la température normale, de sorte que la substance formant support solide non seulement emmagasine et conserve les substances de sapidité et/ou odorantes mais les restitue de façon contrôlée pendant la durée de conservation, et la substance de sapidité et/ou odorante (3) au moins au nombre de un liée dans la substance formant support solide étant, par rapport au produit, disposée d'au moins une des façons: sur le produit (1) ou bien suffisamment près à proximité immédiate du produit (1) pour qu'une quantité suffisante de substance de sapidité et/ou odorante puisse parvenir au produit.

11. Système selon la revendication 10,
**caractérisé en ce que**
l'enveloppe comprend du papier, du poly(éthylène) (15), en particulier du poly(éthylène) modifié, du poly(éthylène) basse densité ou du poly(éthylène) haute densité, ou une feuille de silicone, et en particulier un solvant polaire ou non polaire étant ajouté à la substance de sapidité et/ou odorante (3) pour assurer le passage des substances d'aromatisation à travers l'enveloppe.

12. Système selon la revendication 10 ou la revendication 11,
**caractérisé en ce que**
la substance formant support solide avec au moins une substance de sapidité et/ou odorante (3) qui lui est liée se présente sous forme de poudre (51), de granulés, de paillettes (41), de perles ou similaires, en particulier sous forme de poudre (51) avec un diamètre extérieur dans la plage de 10 µm à 100 µm, de préférence de 25 µm à 50 µm, ou sous forme de granulés (41), de paillettes ou de perles avec un diamètre extérieur dans la plage allant jusqu'à 10 mm, de préférence jusqu'à 5 mm.

13. Système selon les revendications 10 à 12,
**caractérisé en ce que**
la substance formant support (17) solide avec au moins une substance de sapidité et/ou odorante (3) qui lui est liée est mise en place sur un matériau d'appui ou dans le matériau d'appui, le matériau d'appui étant de préférence un matériau plat ou une partie produite par formage initial ou déformation ou une partie de forme quelconque assemblée à partir de plusieurs éléments, en particulier du papier (22), du carton, une feuille en matière plastique ou en métal, de la céramique, du verre, du non-tissé ou du tissu ainsi qu'un produit de type tissu en étoffe, en verre, en céramique en métal ou en fibres de matière plastique, l'enveloppe étant en particulier imperméable au matériau d'appui, le matériau d'appui pour l'application en surface ou intérieure de la substance formant support avec au moins une substance de sapidité et/ou odorante (3) qui lui est liée étant de préférence le côté intérieur du matériau d'un emballage du produit.

14. Système selon l'une des revendications 10 à 13,
**caractérisé en ce que**
l'enveloppe (15) présente une épaisseur de 0,1 mm à 2 mm, de préférence de 0,2 mm à 0,5 mm et/ou la substance de sapidité et/ou odorante (3) au moins au nombre de un est dissoute dans un solvant non polaire, de préférence dans un solvant isoparaffinique qui présente de préférence un point d'inflammation supérieur à 50 °C, en particulier supérieur à 60 °C, la quantité du solvant rapportée à la quantité totale de solvant et de substance de sapidité et/ou odorante (3) étant de 0,5% à 50%, de préférence de 10%.

15. Système selon l'une des revendications 10 à 14,
**caractérisé en ce que**
le système comprend en outre un emballage (12) qui enveloppe les moyens et le produit, l'emballage (12) comprenant en particulier un matériau imperméable à la substance de sapidité et/ou odorante (3), comme de la cellophane, qui est raccordé de préférence d'un seul tenant à l'emballage (12).
